# EUROPEAN PATENT SPECIFICATION

(11) **EP 3 898 224 B1**
(45) Date of publication and mention of the grant of the patent: **06.07.2022**
(21) Application number: 19839142.7
(22) Date of filing: 20.12.2019
(51) Int. Cl.: B32B 5/02, B32B 5/12, B32B 5/26, B32B 37/06

(54) **COMPOSITE MATERIAL AND METHOD FOR PRODUCING THE SAME**
VERBUNDWERKSTOFF UND HERSTELLUNGSVERFAHREN DAFÜR
MATÉRIAU COMPOSITE ET PROCÉDÉ DE FABRICATION DE CELUI-CI

(30) Priority: 20.12.2018 IT 201800020521
(43) Date of publication of application: 27.10.2021
(73) Proprietor: Leonardo S.p.a., 00195 Roma (IT)
(72) Inventor: GALLO, Nicola, 74023 Grottaglie (TA) (IT); CORVAGLIA, Stefano Giuseppe, 74023 Grottaglie (TA) (IT); PAPPADA', Silvio, 74023 Grottaglie (TA) (IT)
(74) Representative: D'Angelo, Fabio
(86) International application number: PCT/IB2019/061196
(87) International publication number: WO 2020/129007

(56) References cited:
- WO-A1-90/08027
- JP-A- 2012 187 903
- US-A1- 2010 133 261
- US-A1- 2015 231 869
- US-A1- 2018 063 895
- Richard A Flinn ET AL: "Chapter 16 Composites", Engineering Materials and Their Applications, 4th edition., 31 December 2005 (2005-12-31), XP055676040, Retrieved from the Internet: URL:http://in.bgu.ac.il/engn/mater/Documen ts/LaboratoryBriefings/4/Materials%20Scien ce%20and%20Engineering%20introduction%20Ch apter%2015%20Composites%207th%20ed.pdf [retrieved on 2020-03-12]
- N Athanasopoulos ET AL: "Calculation of an equivalent electrical conductivity tensor for multidirectional carbon fiber reinforced materials", Progress in Electromagnetics Research Symposium Proceedings, 19 August 2012 (2012-08-19), XP055705142,

## Description

### TECHNICAL FIELD

The present invention relates to a composite material, in particular for aeronautical applications, to which the following description will make explicit reference, but without any loss of generality.

The present invention also relates to a method for producing the aforementioned composite material.

### PRIOR ART

As is known, composite materials are used in various industrial sectors, including the aviation industry. In particular, fibre-reinforced composite materials, commonly referred to as "pre-impregnated" or "prepreg", are known, which are generally constituted by a semifinished product comprising a resin matrix and reinforcing fibres immersed in the matrix. The fibres can be arranged according to different configurations, for example, in a single direction, in two or more mutually different directions, or can be arranged to form a fabric. The matrix is used to fix the fibres to each other and possibly to other components during production.

Prepregs are generally prepared in the form of tapes and wound in rolls; in order to achieve the desired mechanical properties, prepregs must be subjected to a consolidation process through heat and also often under pressure.

The prepregs mainly used in the aviation industry can have a matrix of a thermosetting material or of a thermoplastic material.

In the first case (thermosetting materials), the matrix is constituted by polymers that, in opportune temperature conditions and/or in the presence of certain substances, transform into rigid, insoluble and infusible materials. This transformation occurs following cross-linkage reactions (a process known as curing, through which polymer chains undergo a reaction that creates bonds between different chains at a reactive functional group level), which take place between the polymer chains with the formation of strong (covalent or ionic) bonds.

Before polymerization, thermosetting materials have characteristics of stickiness. These materials can therefore be used to create stratifications, by placing different layers one on top of the other, with an opportune sequence or orienting of the different layers. The stratifications are then subjected to a temperature and pressure cycle (in a vacuum bag and in autoclave, using ovens, moulding presses, etc.) which polymerizes the material, raising the molecular weight and inducing the formation of bonds between the macromolecules (cross-linking), thus transforming it into a material with structural characteristics and mechanical properties suitable for its intended application.

Some thermosetting polymers are cross-linked by just heat or through a combination of pressure and heat, while others can be cross-linked through chemical reactions at room temperature (cold cross-linking).

The layering can be accomplished with automated methods, which provide significant advantages in terms of cost, productivity and repetitiveness. For flat or moderately curved layering, an Automated Tape Laying (ATL) device is used. Recently, a technique has become popular that allows layering on curved or even closed (cylindrical) surfaces using prepregs tapes of rather small width (known as slits); this technique is called Fibre Placement (FP).

In the second case (thermoplastic materials), the matrix resin has a high molecular weight and therefore, on one hand, it does not need to undergo a polymerization cycle, and on the other, does not have characteristics of stickiness.

In a first approximation, a thermoplastic matrix prepreg can be considered a manufactured product in its final state formed by a single lamina. To be able to form a laminate, it is necessary to heat it so as to cause the melting of at least the contact surfaces of its constituent laminae or layers of thermoplastic prepreg, compress it under pressure and then let it cool. The temperature to be reached for melting is the glass transition temperature T_{g} for amorphous thermoplastics, and the melting point temperature T_{f} for semi-crystalline thermoplastics.

Currently, the techniques of automated tape laying (ATL) and fibre placement (FP) are only used for composite parts with a thermosetting matrix. These are also conceptually possible techniques for thermoplastic-matrix prepregs, but have some additional technological requirements; in fact, in this case, the apparatus for producing a laminate based on thermoplastic prepregs must also provide the heat for reaching a temperature (which, depending on the materials, might be excessively high) such as to melt the resin and thus obtain adhesion between the various layers that will constitute the laminate; in addition, for semi-crystalline thermoplastics, cooling that is too rapid might cause amorphisation of the part, with consequent loss of performance characteristics. In compensation, if these problems were solved, the ATL and FP techniques would enable achieving finished parts without a further autoclave process, with a significant reduction in the production costs of the parts.

As previously explained, the processes of consolidation of the prepregs and of bonding the various prepreg layers that form the final component usually take place in an autoclave, in ovens or moulding presses. In the case of very large components, such as, for example, structural components in the aviation sector, naval sector, etc., the known consolidation and bonding processes are excessively expensive and can create numerous undesired constraints.

The need has thus been felt to develop techniques that allow achieving in-situ consolidation and bonding of parts in a composite material, especially when these are very large.

One of the most promising techniques for composite materials with a thermoplastic matrix is consolidation and bonding by electromagnetic induction, which, unlike other techniques, such as, for example gluing, does not use filler materials, but only the overlapping of two edges of two parts to be bonded (also known as adherends), at least one being electrically conductive, kept in contact with each other.

According to this technique, an inductor, for example a coil, generates a variable electromagnetic field, which in turn causes the induction of parasitic currents in the areas of the adherends to be bonded, at least one of which is electrically conductive; by the Joule effect, these parasitic currents heat the matrix of the adherends, bringing it to the melting or softening point; then, when the adherends have been brought to the temperature considered ideal, mechanical pressure can be applied, known as the consolidation pressure, suitable for inducing the adhesion of the adherends.

In traditional induction bonding systems, bearing in mind a dependence related to the square of the distance from the inductor, the heating action due to parasitic currents tends to be concentrated on the surface directly exposed to the inductor. A consequently steep temperature gradient is thus produced, which causes surface melting of the matrix rather than of the area of adhesion of the adherends: this negatively impacts the quality of the bond and of the mechanical characteristics of the bond thus obtained.

Furthermore, the direction of this temperature gradient, in the direction of the thickness, results in a maximum temperature on the surface facing the inductor and a minimum temperature on the bonding surface. Therefore, in order to reach the ideal temperature on the adhesion surface of the adherends, a temperature in the outermost areas will be excessive and this can lead to degradation of the composite's matrix. The presence of this gradient consequently limits the possible thickness of the adherends quite significantly.

Similarly, there is a large concentration of currents on the edges of the parts to be bonded and, therefore, temperature gradients that are often unacceptable form on them, which can again lead to degradation of the material.

In any case, the temperatures that are reached on the adhesion surface are too low (generally 60 - 70°C) with respect to that required for forming the bond (approximately 350°C).

Ultimately, the main drawback of in-situ consolidation and bonding techniques of composite materials by electromagnetic induction lies in the difficulty of optimizing the temperature distribution through the thickness of the bond.

WO90/08027A1 discloses a composite material suitable electromagnetic induction bonding and comprising a core layer comprising a plurality of layers of unidirectional carbon fibre reinforced PEEK, and a second layer bonded to the first layer and made of a random oriented carbon fibre mat.

### DESCRIPTION OF INVENTION

One object of the present invention is to produce a composite material that enables overcoming the above-described drawbacks.

According to the present invention, a composite material is provided, in particular for aeronautical applications, as claimed in claim 1 and in its dependent claims.

The present invention also relates to a method as claimed in claims 9 and 10 for producing the aforementioned composite material.

### BRIEF DESCRIPTION OF DRAWINGS

The present invention will now be described with reference to the accompanying drawings, which show two different non-limitative embodiments, in which:
- Figure 1 shows a schematic perspective view of two layers of starting materials to be joined to form a first embodiment of a lamina of composite material according to the present invention;
- Figure 2 shows a schematic view, highly enlarged and in section along line II-II of Figure 1;
- Figure 3 is a schematic view, in section, similar to Figure 2 and represents the lamina of composite material obtained by the joining of two layers of starting materials of Figures 1 and 2;
- Figure 4 is a perspective view of a layout showing a possible implementation of a bonding operation of two laminae of composite material of the type shown in Figure 3;
- Figure 5 shows a schematic view, highly enlarged and in section along line V-V of Figure 4;
- Figure 6 is a photograph, obtained using a microscope, of a part of the two laminae of composite material bonded together and shown in Figures 4 and 5;
- Figure 7 shows a highly enlarged detail of Figure 6;
- Figure 8 shows a temperature-time diagram obtainable at the bonding interface between the two laminae of composite material of Figures 4 to 7 during the bonding operation in Figure 4;
- Figure 9 shows a temperature-time diagram obtainable at the bonding interface between two laminae of composite material of a known type;
- Figure 10 shows a histogram in which the moduli of elasticity for laminae of composite material according to the present invention and laminae of composite material of a different type are compared;
- Figure 11 shows a histogram in which the tensile strengths calculated for laminae of composite material according to the present invention and laminae of composite material of a different type are compared;
- Figure 12 shows the same histogram of Figure 10 where one of the moduli of elasticity calculated for the different laminae of composite material is set to 1;
- Figure 13 shows the same histogram of Figure 11 where one of the tensile strengths calculated for the different laminae of composite material is set to 1;
- Figure 14 shows a histogram that compares the thicknesses of the different laminae for which the values of modulus of elasticity and tensile strength have been calculated in the histograms of Figures 10 to 13;
- Figure 15 is a figure similar to Figure 2 and shows a schematic view, in section and on a highly enlarged scale, of two layers of starting materials destined to be joined to form a second embodiment of a lamina of composite material according to the present invention; and
- Figure 16 is a schematic view, in section, similar to Figure 3 and shows the lamina of composite material obtained from the joining of the two layers of starting materials of Figure 15.

### PREFERRED EMBODIMENTS OF THE INVENTION

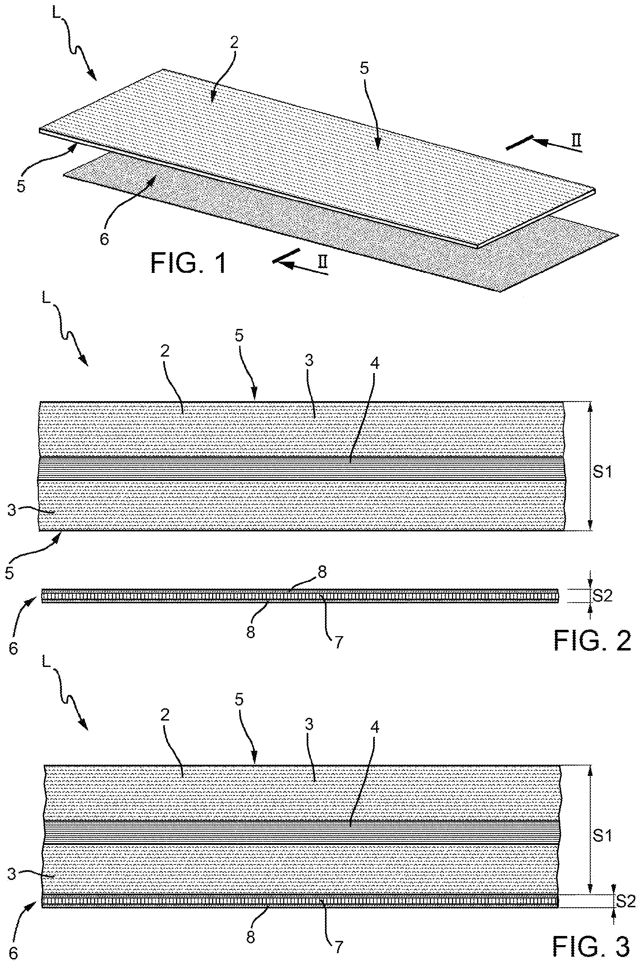

Figures 1 to 3 schematically show a first embodiment of a lamina L of composite material, produced according to the present invention and, in particular, designed to be used in aeronautic applications.

The lamina L of composite material comprises a first pre-impregnated layer 2 having a resin-based matrix 3, reinforced with fibres 4 or with a fibre material to give the layer 2 predefined mechanical properties.

It should be noted that the expression "-based" or "based on" means that the matrix 3 can, in addition to the resin, also comprise commonly used additives such as, for example, fillers, stabilizers, etc.

The matrix 3 is preferably based on a semi-crystalline thermoplastic resin having a predetermined melting point T_{f}. This semi-crystalline thermoplastic resin is, for example, polyether ether ketone, or PEEK, which has a melting point temperature T_{f} of approximately 340°C. In alternative, this semi-crystalline thermoplastic resin can, for example, be polyetherketoneketone, or PEKK, which has a melting point temperature T_{f} of approximately 370°C.

The matrix 3 can also be based on an amorphous thermoplastic resin having a predetermined glass transition temperature T_{g}. This amorphous thermoplastic resin is, for example, polyetherimide, or PEI, which has a glass transition temperature of approximately 215°C.

The fibres 4 are preferably carbon fibres and are arranged in one or more unidirectional layers.

Carbon fibres 4 have an areal weight preferably comprised between 100 and 300 g/m².

The fibres 4 of the layer 2 are unidirectional; in particular, the unidirectional fibres 4 are used to unidirectionally reinforce the stressed parts of the lamina L, arranging them along the load path.

In alternative, other types of fibres known in the aviation sector can be used for the layer 2, such as, for example, glass fibres or a combination of glass and carbon fibres.

The fibres 4 are preferably continuous or can have a length comprised between 1 mm and 40 cm.

As visible in Figures 1 and 2, the layer 2 is delimited by respective opposite faces 5, parallel to each other; the layer 2 has a thickness S1, in a direction orthogonal to the faces 5, which is smaller with respect to the sizes (length and width) of the faces 5.

The lamina L of composite material further comprises a second layer 6 of magnetic field intensifier material, superimposed and joined to one of the faces 5 of layer 2; electrically conductive fibres 7 having equivalent electrical resistivity and less than 600 µΩm, preferably less than 200 µΩm and even more preferably less than 100 µΩm,so as to facilitate localized heating through electromagnetic induction, are dispersed in layer 6 in at least two directions with different orientations and parallel to the faces 5.

For the significance and calculation of the equivalent electrical resistivity (inverse of equivalent electrical conductivity) of a composite material, reference can, for example, be made to the following publication:
"Calculation of an equivalent electrical conductivity tensor for multidirectional carbon fiber reinforced materials", 19 August 2012, authors N. Athanasopoulos and V. Kostopoulos.

In the example shown, the layer 6 comprises a matrix 8 based on a semi-crystalline thermoplastic resin, identical to the resin of the matrix 3 of layer 2 or compatible with the latter, in which the fibres 7 are dispersed. More specifically, the fibres 7 are in carbon and are arranged in the matrix 8 in a random manner in a plurality of directions, some of which extend parallel to the faces 5, while others extend transversely to the faces 5.

The fibres 7 extending transversely to the faces 5 have an equivalent electrical resistivity greater than 2,000 µΩm.

The carbon fibres 7 have an areal weight comprised between 10 and 100 g/m².

The carbon fibres 7 are preferably obtained through the oxidation and pyrolysis of polyacrylonitrile (PAN).

Alternatively, the carbon fibres 7 could be obtained through the distillation of carbon-based materials such as, for example, plants, raw oil and carbon (PITCH); in this case, a viscoelastic material composed of aromatic hydrocarbons.

The fibres 7 can also be made in another electrically conductive material, for example, a metal material.

The fibres 7 are preferably continuous or can have a length comprised between 1 mm and 40 cm.

Advantageously, the layer 6 is in the form of a veil or film. The layer 6 has, in a direction orthogonal to the faces 5, a thickness S2 comprised between 1/10 and 1/100 of the thickness S1 of the layer 2.

The layers 2 and 6 are joined by compression moulding and preferably by continuous compression moulding. This joining can be performed in a known manner in a hot press (known and not shown) or by making the layers 2 and 6 to be joined pass between respective pairs of hot rollers (known and not shown) in a continuous lamination-like process. The temperature at which the joining process of the layers 2 and 6 takes place depends on the kind of thermoplastic matrix used; in the case of semi-crystalline thermoplastic matrices it is higher than the melting point T_{f}, while in the case of amorphous thermoplastic matrices it is greater than the glass transition temperature T_{g}.

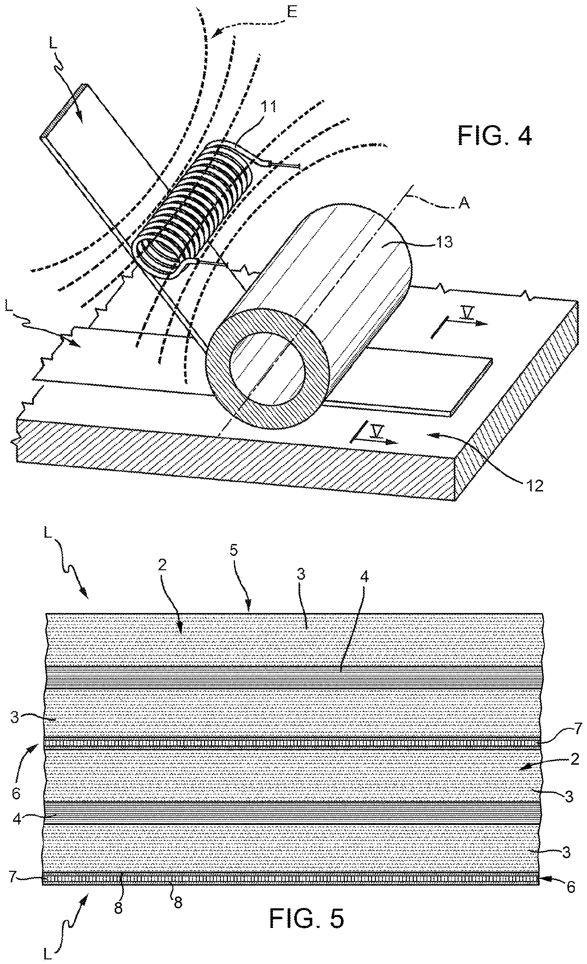

Figure 4 schematically shows the essential means of implementing a bonding operation of two laminae L of composite material.

During this operation, an inductor 11 (known and only schematically shown), for example an electric coil, is fed with electric current so as to generate a variable electromagnetic field E (the flux lines of which are schematically indicated by broken lines in Figure 4). At the same time, a new lamina L is progressively laid on another already present lamina L, arranged on a supporting surface 12, or, alternatively, on an upper lamina L of a series of already joined or bonded laminae L arranged on the supporting surface 12.

The step of laying the new lamina L is performed by one or more rollers 13 (only one of which is shown in Figure 4), having axes A parallel to the laminae L on which they operate, rotating about the axes A and advancing parallel to the supporting surface 12.

The electromagnetic field E induces parasitic currents essentially in the layer 6 of the new lamina L which is then joined to the other lamina (e) L lying on the supporting surface 12.

Since the fibres 7 of the layer 6 are electrically conductive and are dispersed in the matrix 8 in at least two different directions, actual "electrical circuits" are created, at least inside layer 6 of the new lamina L, which heat, by the Joule effect, the matrix 8 of layer 6 and the adjacent lamina L.

The localized heating is very efficient and enables reaching temperatures locally, i.e. in the layer 6 and in the areas of the matrices 3 adjacent to this layer, that exceed the melting point temperature T_{f} or the glass transition temperature T_{g}.

Instead, in the areas of the matrices 3 furthest away from layer 6, the temperature remains decidedly below the melting point temperature T_{f} of the resin forming these matrices 3.

The passage of the roller 13, or rollers 13, takes place after the induction of parasitic currents in the new lamina L to be bonded to the other lamina (e) L arranged on the surface of the supporting surface 12.

In this way, pressure is progressively exerted on the areas of the laminae L to be bonded, after the softening obtained at the interface between them following electromagnet induction and during the cooling of these areas.

Preferably, the roller(s) 13 is/are kept cold so as to progressively remove residual heat from the laminae L in the bonding step.

At the end of the bonding operation, the result is that schematized in Figure 5.

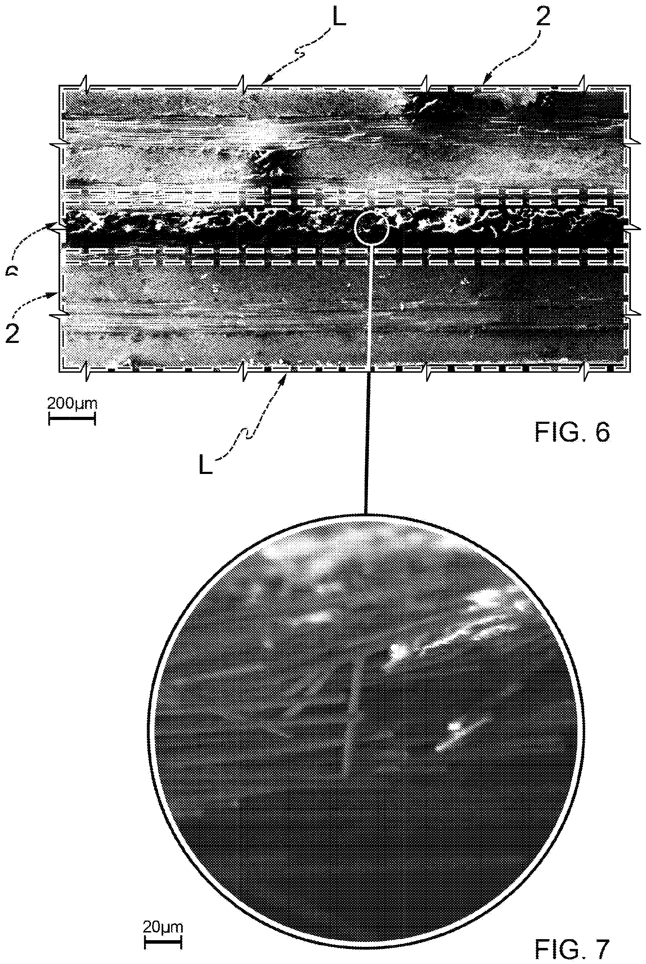

Figures 6 and 7 show microscope images of the bonding areas of the laminae L, each formed by:
- a layer 2 of PEKK with unidirectional carbon fibres 4 having an areal weight of 200 g/m²; and
- a layer 6 of VEIL with a matrix 8 in PEKK and carbon fibres 7 with an areal weight of 8 g/m² dispersed in a random manner in all directions.

As can be seen, the fibres 7 have a substantially isotropic distribution in the matrix 8 of layer 6, with some of the fibres 7 directed transversely with respect to the faces 5 of the layer 2. This enables achieving high resistance/toughness of the bonded material.

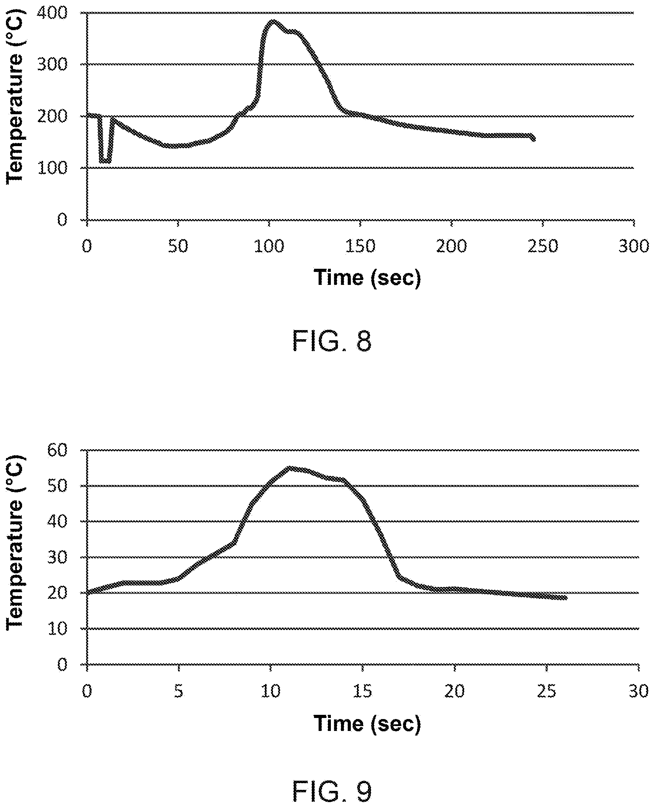

Figures 8 and 9 show two temperature/time diagrams that compare, during a bonding operation by electromagnetic induction, the behaviour of:
- two laminae L of composite material according to the present invention; and
- two laminae of composite material of a known type. In particular, the diagram of Figure 8 refers to two laminae L, each of which is formed by:
- a layer 2 di PEKK with unidirectional carbon fibres having an areal weight of 200 g/m²; and by
- a layer 6 of VEIL with matrix 8 in PEKK and carbon fibres 7 with an areal weight of 8 g/m² dispersed in a random manner in all directions.

The diagram in Figure 9 refers instead to two laminae of composite material of a known type, with matrix in PEKK and unidirectional carbon fibres having an areal weight of 200 g/m².

As can be observed, in the first case (Figure 8), temperatures close to 400°C are reached for a few tens of seconds (25 - 30 seconds).

In the second case (Figure 9), the maximum temperature reached is below 60°C.

The applicant has observed that, due to the adoption of a thickness for layer 6 comprised between 1/10 and 1/100 of the thickness of the layer 2, it is possible to achieve, on one hand, substantial preservation in the lamina L of the mechanical properties determined by the layer 2 and, on the other, extremely easy in-situ consolidation and bonding of the material by electromagnetic induction.

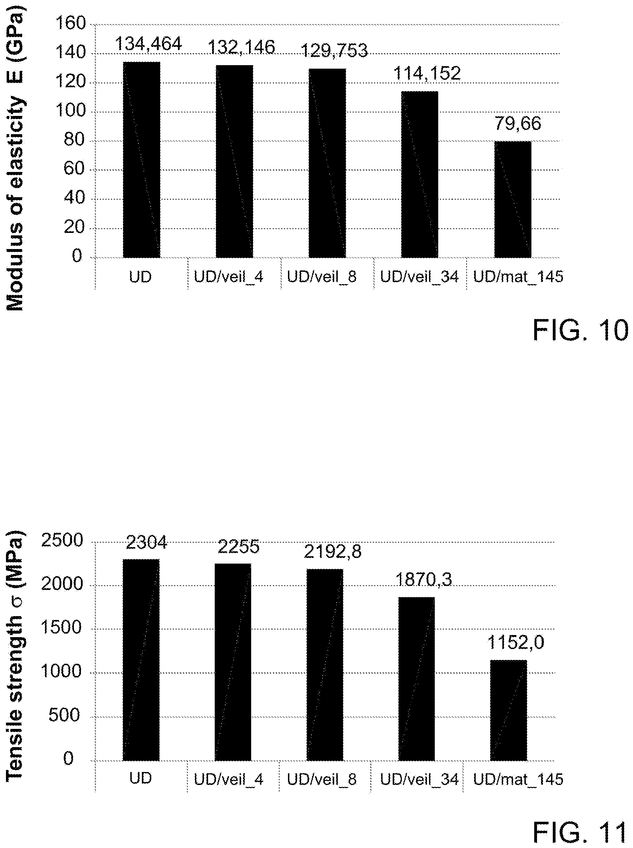

Figures 10 to 14 show experimental results obtained by the applicant, in terms of mechanical properties and thickness, when comparing the following types of laminae:
- unidirectional PEKK/carbon lamina (APC (PEKK-FC)_ AS4 (Wm%=34%, areal weight or grams per square metre = 145 g/m²), hereinafter referred to as UD and corresponding to an example of a lamina not forming part of the present invention and constituted by just layer 2;
- lamina according to the present invention obtained by coupling the previous UD lamina (layer 2) with a veil layer 6 having an areal weight of 4 g/m² (hereinafter referred to as UD/veil_4);
- lamina according to the present invention obtained by coupling the previous UD lamina (layer 2) with a veil layer 6 having an areal weight of 8 g/m² (hereinafter referred to as UD/veil_8);
- lamina not forming part of the present invention and obtained by coupling the previous UD lamina (layer 2) with a veil layer 6 having an areal weight of 34 g/m² (hereinafter referred to as UD/veil_34); and
- lamina not forming part of the present invention and obtained by coupling the previous UD lamina (layer 2) with a mat layer 6 having an areal weight of 145 g/m² (hereinafter referred to as UD/mat_145).

The above-indicated veil and mat have a semi-crystalline thermoplastic resin based matrix, identical to the resin of the matrix of the UD lamina.

The impregnation level of the above-indicated veil and mat is 34% by weight.

The fibres used in the above-indicated veil and mat are in carbon and are arranged in the respective matrices in a random manner in all directions.

The mechanical properties of the different types of laminae described above have been determined in terms of modulus of elasticity E and tensile strength σ; both quantities have been calculated in the direction parallel to the unidirectional fibres in layer 2 or in the UD lamina.

The thickness of the different laminae considered has been calculated in the direction orthogonal to the laminae and is listed in the table below:

| **Lamina** | **UD thickness (mm)** | **Veil or Mat thickness (mm)** | **Coupled thickness (mm)** |
|---|---|---|---|
| UD | 0.138 | - | 0.138 |
| UD/veil 4 | 0.138 | 0.004 | 0.141 |
| UD/veil 8 | 0.138 | 0.008 | 0.145 |
| UD/veil 34 | 0.138 | 0.032 | 0.170 |
| UD/mat 145 | 0.138 | 0.138 | 0.276 |

Instead, the table below lists the values of the modulus of elasticity E and tensile strength σ calculated for the above-defined laminae.

| **Lamina** | **Modulus of elasticity E (GPa)** | **Tensile strength σ (MPa)** |
|---|---|---|
| UD | 134.464 | 2304 |
| UD/veil 4 | 132.146 | 2255 |
| UD/veil 8 | 129.753 | 2192 |
| UD/veil 34 | 114.152 | 1870 |
| UD/mat 145 | 79.66 | 1152 |

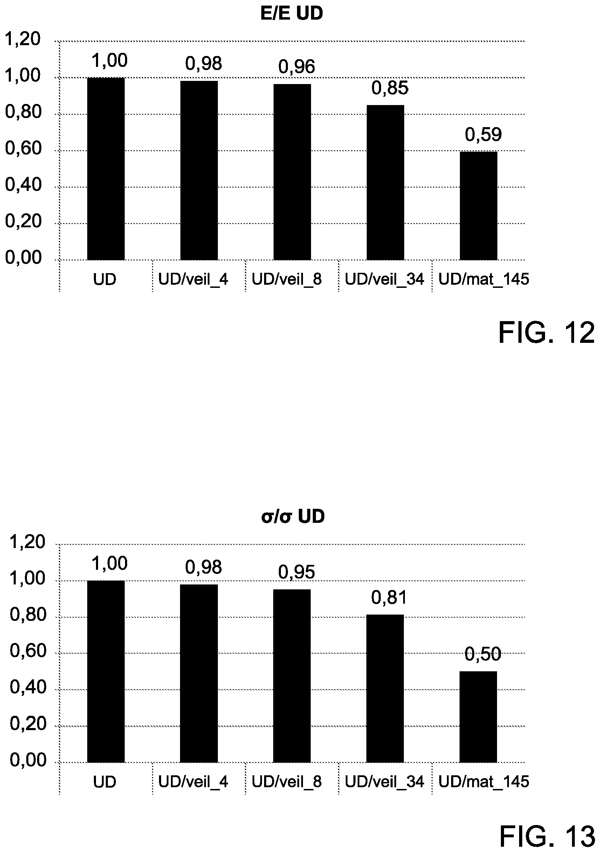

The results of this table are summarized as histograms in Figures 10 and 11 and, in normalized form, in Figures 12 and 13.

In particular, as can be observed both from the previous tables and from Figures 10 and 11, the deterioration of the mechanical properties starts to be already appreciable with a thickness of the layer 6 equal to 23% of the thickness of the layer 2 and becomes very marked in the case where the two layers 2, 6 have the same thickness.

The histograms of Figures 12 and 13 refer to the same results shown in Figures 10 and 11, but where the modulus of elasticity E and tensile strength σ of the UD lamina are used as the denominator in the ratio with the values of the corresponding quantities calculated for the other types of lamina.

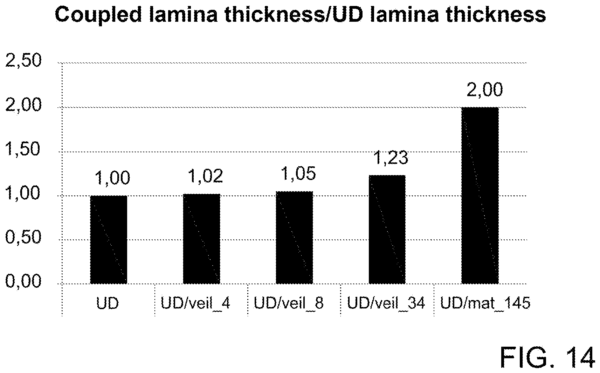

Figure 14 shows the ratios between thicknesses of the different types of lamina and the thickness of the UD lamina taken as reference.

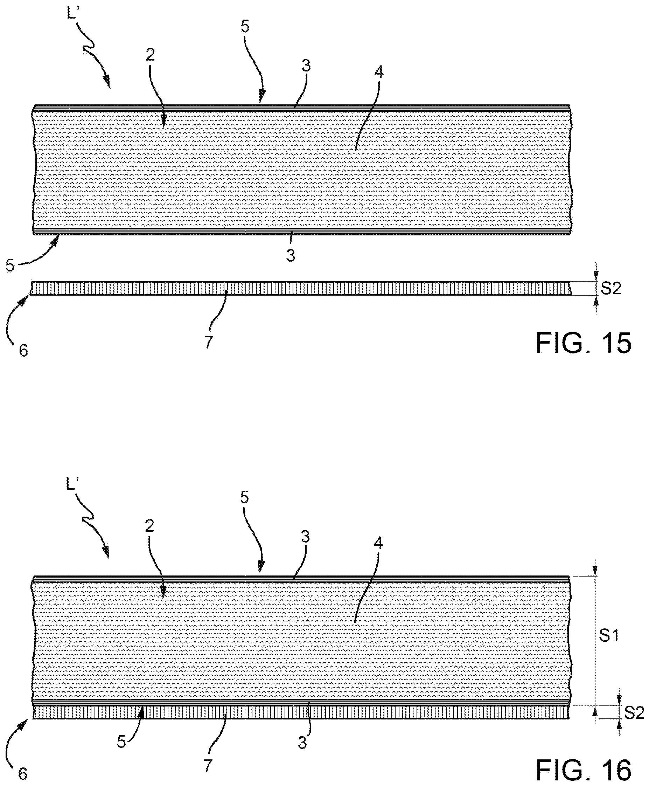

Figures 15 and 16 show a lamina of composite material produced according to a different embodiment of the present invention and indicated as a whole by L'; the lamina L' will be described below only with regard to how it differs from lamina L, where possible, indicating parts that are identical, corresponding or equivalent to parts already described with the same reference numerals.

In particular, lamina L' of composite material differs from lamina L basically in that it comprises a magnetic field intensifier layer 6' without a resin matrix.

In greater detail, the layer 6' can be a carbon veil in which the fibres 7 extend in at least two directions having different orientations, preferably in at least three directions of which one is transversal to the faces 5 of the layer 2; in this case, the fibres 7 are held together to form the veil by an adhesive or binder, for example PVA (polyvinyl alcohol) or other substances chemically compatible with the matrix 3 of the layer 2.

Alternatively, the layer 6' could be a fabric with the fibres 7 extending in at least two directions with different orientations.

According to a further alternative, the layer 6' could be a non-woven fabric, for example of the Optiveil^{®} type.

From an examination of the characteristics of the composite material produced according to the principles of the present invention, the advantages that can achieved therewith are evident.

In particular, as previously stated, since the fibres 7 of the layer 6, 6' are electrically conductive and are arranged in at least two directions with different orientations, it is possible to generate actual "electrical circuits" by electromagnetic induction inside at least layer 6, 6'; the creation of these circuits enables achieving effective heating, by the Joule effect, of the layer 6, 6' and of the adjacent resin matrices 3, reaching the melting point temperature T_{f} of the resin precisely at the bonding interface of two different laminae L, L'.

In practice, reaching the melting point temperature T_{f} of the resin at the aforementioned interface does not require generating even higher temperatures in the areas of the resin closest to the inductor 11, with the consequent possible degradation of the matrix of the composite material.

As shown in the diagrams of Figures 8 and 9, the temperatures reached at the bonding interface between two laminae L, L' according to the present invention during an operation of in-situ consolidation and bonding by electromagnetic induction are at least 7 times higher than those reached in the case of composite materials of a known type.

Precisely for the reasons set out above, the applicant has also measured a bonding speed between two laminae L, L' according to the present invention ten times higher than the bonding speed of traditional prepregs.

Since the thickness S2 of the layer 6, 6' is comprised between 1/10 and 1/100 of the thickness S1 of layer 2, easy in-situ consolidation and bonding of the material according to the present invention is achieved practically without penalising the mechanical properties given to the laminae L, L' by the orientation and arrangement of the carbon fibres of the layer 2. Finally, it is clear that modifications and variants can be made to the composite material as well as to the method for producing this composite material set forth herein, without departing from the scope defined in the claims.

## Claims

1. A composite material, in particular for aeronautical applications, comprising:
- a first pre-impregnated layer (2) having a thermoplastic resin-based matrix (3) reinforced with unidirectional fibres (4) to give the first layer (2) predefined mechanical properties; and
- a second layer (6, 6') of magnetic field intensifier material, superimposed on a face (5) of said first layer (2) and joined to the first layer (2) along said face (5); said second layer (6, 6') comprising electrically conductive fibres (7), preferably of carbon, dispersed in the second layer (6, 6') in at least two directions with different orientations so as to facilitate localized heating by electromagnetic induction; said first and second layers (2; 6, 6') joined together defining a lamina (L, L') of very easily heated composite material;
**characterized in that**, with reference to a direction orthogonal to said face (5), said second layer (6, 6') has a thickness (S2) comprised between 1/10 and 1/100 of the thickness (S1) of said first layer (2) so as to make said lamina (L, L') suitable for being consolidated and in-situ bonded to another said lamina (L' , L') of the same type by electromagnetic induction.

2. The composite material according to claim 1, wherein said electrically conductive fibres (7) of said second layer (6, 6') have equivalent electrical resistivity, parallel to said face (5), of less than 600 µΩm, preferably less than 200 µΩm and even more preferably less than 100 µΩm.

3. The composite material according to any one of the preceding claims, wherein one of said directions in which said electrically conductive fibres (7) are dispersed in said second layer (6, 6') is transversal to said face (5).

4. The composite material according to any one of the preceding claims, wherein said electrically conductive fibres (7) are dispersed in said second layer (6, 6') in at least three directions with mutually different orientations, of which at least one of said directions is transversal to said face (5).

5. The composite material according to any one of the preceding claims, wherein said electrically conductive fibres (7) of said second layer (6, 6'):
- are held together by a resin-based matrix (8), identical or compatible with the resin of the matrix (5) of said first layer (2); or
- are held together to form a veil by an adhesive or binder.

6. The composite material according to claim 5, wherein said electrically conductive fibres (7) of said second layer (6, 6') form a fabric, preferably a non-woven fabric.

7. The composite material according to any one of the preceding claims, comprising a further first layer (2), and wherein said first layers (2) are arranged on opposite sides of said second layer (6, 6') and are bonded by electromagnetic induction through at least the second layer (6, 6').

8. The composite material according to any one of claims 1 to 6, comprising a plurality of said laminae (L, L') bonded together, wherein the first layers (2) are alternated with respective said second layers (6, 6') and wherein the first layers (2) of each pair of consecutive first layers (2) are arranged on opposite sides of a respective said second layer (6, 6') and are bonded by electromagnetic induction through at least said second layer (6, 6').

9. A method for producing a composite material according to any one of the preceding claims, in which one said first layer and one said second layer (2; 6, 6') are joined together to form one said lamina (L, L') of super-weldable composite material by hot compression moulding, preferably by continuous compression moulding, by passing said first and second layers (2; 6, 6') between at least one pair of hot rollers.

10. The method according to claim 9, comprising the operation of bonding together at least two said laminae (L, L') of composite material through the following steps:
- progressively spreading a first one of said laminae (L, L') onto a second one of said laminae (L, L') lying on a support (12);
- inducing a parasitic electrical current at least in said second layer (6, 6') of said first lamina (L, L') by means of an inductor (11) so as to produce localized heating of the areas of said first and second laminas (L, L') adjacent to said second layer (6, 6') of said first lamina (L, L'); and
- applying, after said inducing step, a pressure action on said first lamina (L, L') to make it adhere to said second lamina (L, L') during the cooling of the laminae (L, L').

## Patentansprüche

1. Verbundmaterial, insbesondere für Luftfahrtanwendungen, das Folgendes umfasst:
- eine erste vorimprägnierte Lage (2), die einen Grundstoff (3) auf Basis eines thermoplastischen Harzes hat, die mit unidirektionalen Fasern (4) verstärkt ist, um der ersten Lage (2) zuvor festgelegte mechanische Eigenschaften zu verleihen; und
- eine zweite Lage (6, 6') eines Materials, das ein Magnetfeld verstärkt, die auf eine Fläche (5) der ersten Lage (2) gelegt und längs der Fläche (5) mit der ersten Lage (2) verbunden ist; wobei die zweite Lage (6, 6') elektrisch leitfähige Fasern (7), vorzugsweise aus Kohlenstoff, umfasst, die in der zweiten Lage (6, 6') in wenigstens zwei Richtungen mit unterschiedlichen Orientierungen verteilt sind, um ein lokales Erhitzen durch elektromagnetische Induktion zu ermöglichen; wobei die erste und die zweite Lage (2; 6, 6'), die miteinander verbunden sind, eine Schicht (L, L') aus sehr leicht erhitzbarem Verbundmaterial definieren;
**dadurch gekennzeichnet, dass** in Bezug auf eine Richtung senkrecht zur Fläche (5) die zweite Lage (6, 6') eine Dicke (S2) im Bereich zwischen 1/10 und 1/100 der Dicke (S1) der ersten Lage (2) hat, so dass die Schicht (L, L') verfestigt und mit einer weiteren Schicht (L, L') des gleichen Typs durch elektromagnetische Induktion in situ verbunden werden kann.

2. Verbundmaterial nach Anspruch 1, wobei die elektrisch leitfähigen Fasern (7) der zweiten Lage (6, 6') einen entsprechenden elektrischen Widerstand parallel zur Fläche (5) von weniger als 600 µΩm, vorzugsweise weniger als 200 µΩm und noch stärker bevorzugt weniger als 100 µΩm haben.

3. Verbundmaterial nach einem der vorhergehenden Ansprüche, wobei eine der Richtungen, in der die elektrisch leitfähigen Fasern (7) in der zweiten Lage (6, 6') verteilt sind, transversal zur Fläche (5) ist.

4. Verbundmaterial nach einem der vorhergehenden Ansprüche, wobei die elektrisch leitfähigen Fasern (7) in der zweiten Lage (6, 6') in wenigstens drei Richtungen mit jeweils unterschiedlichen Orientierungen verteilt sind, wobei wenigstens eine der Richtungen transversal zur Fläche (5) verläuft.

5. Verbundmaterial nach einem der vorhergehenden Ansprüche, wobei die elektrisch leitfähigen Fasern (7) der zweiten Lage (6, 6'):
- durch einen Grundstoff (8) auf Harzbasis zusammengehalten werden, die mit dem Harz des Grundstoffs (5) der ersten Lage (2) identisch oder kompatibel ist; oder
- durch einen Klebstoff oder ein Bindemittel zusammengehalten werden, um einen Schleier zu bilden.

6. Verbundmaterial nach Anspruch 5, wobei die elektrisch leitfähigen Fasern (7) der zweiten Lage (6, 6') ein Gewebe, vorzugsweise ein Vlies bilden.

7. Verbundmaterial nach einem der vorhergehenden Ansprüche, das eine weitere erste Lage (2) umfasst, und wobei die ersten Lagen (2) auf entgegengesetzten Seiten der zweiten Lage (6, 6') angeordnet sind und durch elektromagnetische Induktion durch wenigstens die zweite Lage (6, 6') miteinander verbunden werden.

8. Verbundmaterial nach einem der Ansprüche 1 bis 6, das mehrere Schichten (L, L'), die miteinander verbunden sind, umfasst, wobei sich die ersten Lagen (2) in Bezug auf die zweiten Lagen (6, 6') abwechseln und wobei die ersten Lagen (2) jedes Paars aufeinanderfolgender erster Lagen (2) auf entgegengesetzten Seiten einer entsprechenden zweiten Lage (6, 6') angeordnet sind und durch elektromagnetische Induktion durch wenigstens die zweite Lage (6, 6') miteinander verbunden werden.

9. Verfahren zum Herstellen eines Verbundmaterials nach einem der vorhergehenden Ansprüche, wobei eine erste Lage und eine zweite Lage (2; 6, 6') miteinander verbunden werden, um eine Schicht (L, L') eines hervorragend schweißbaren Verbundmaterials durch Formpressen, vorzugsweise durch kontinuierliches Formpressen zu bilden, indem die erste und die zweite Lage (2; 6, 6') zwischen wenigstens einem Paar Heißwalzen hindurchlaufen.

10. Verfahren nach Anspruch 9, das den Vorgang des Verbindens von wenigstens zwei Schichten (L, L') aus Verbundmaterial durch die folgenden Schritte umfasst:
- fortschreitendes Ausbreiten einer ersten Schicht (L, L') auf einer zweiten Schicht (L, L'), die auf einem Träger (12) liegt;
- Induzieren eines elektrischen Fremdstroms wenigstens in der zweiten Lage (6, 6') der ersten Schicht (L, L') mittels einer Induktionsspule (11), um eine lokale Erhitzung der Bereiche der ersten und der zweiten Schicht (L, L') angrenzend an die zweite Lage (6, 6') der ersten Schicht (L, L') zu erzeugen; und
- nach dem Induktionsschritt Ausüben einer Druckeinwirkung auf die erste Schicht (L, L'), damit sie während des Abkühlens der Schichten (L, L') an der zweiten Schicht (L, L') haftet.

## Revendications

1. Matériau composite, en particulier pour applications aéronautiques, comprenant :
- une première couche pré-imprégnée (2) présentant une matrice à base de résine thermoplastique (3) renforcée de fibres unidirectionnelles (4) pour donner à la première couche (2) des propriétés mécaniques prédéfinies ; et
- une seconde couche (6, 6') de matériau intensificateur de champ magnétique, superposée sur une face (5) de ladite première couche (2) et reliée à la première couche (2) le long de ladite face (5) ; ladite seconde couche (6, 6') comprenant des fibres électroconductrices (7), de préférence en carbone, dispersées dans la seconde couche (6, 6') dans au moins deux directions avec des orientations différentes de manière à faciliter un chauffage localisé par induction électromagnétique ; lesdites première et seconde couches (2 ; 6, 6') reliées l'une à l'autre définissant une lame (L, L') de matériau composite très facilement chauffé ;
**caractérisé en ce que**, en référence à une direction orthogonale à ladite face (5), ladite seconde couche (6, 6') présente une épaisseur (S2) comprise entre 1/10 et 1/100 de l'épaisseur (S1) de ladite première couche (2) de manière à rendre ladite lame (L, L') appropriée pour être consolidée et liée in-situ à une autre dite lame (L', L') du même type par induction électromagnétique.

2. Matériau composite selon la revendication 1, dans lequel lesdites fibres électroconductrices (7) de ladite seconde couche (6, 6') présentent une résistivité électrique équivalente, parallèle à ladite face (5), de moins de 600 µΩm, de préférence de moins de 200 µΩm et encore plus préférablement de moins de 100 µΩm.

3. Matériau composite selon l'une quelconque des revendications précédentes, dans lequel l'une desdites directions dans lesquelles lesdites fibres électroconductrices (7) sont dispersées dans ladite seconde couche (6, 6') est transversale à ladite face (5).

4. Matériau composite selon l'une quelconque des revendications précédentes, dans lequel lesdites fibres électroconductrices (7) sont dispersées dans ladite seconde couche (6, 6') dans au moins trois directions avec des orientations mutuellement différentes, dont au moins une desdites directions est transversale à ladite face (5) .

5. Matériau composite selon l'une quelconque des revendications précédentes, dans lequel lesdites fibres électroconductrices (7) de ladite seconde couche (6, 6') :
- sont maintenues ensemble par une matrice à base de résine (8), identique ou compatible avec la résine de la matrice (5) de ladite première couche (2) ; ou
- sont maintenues ensemble pour former un voile par un adhésif ou un liant.

6. Matériau composite selon la revendication 5, dans lequel lesdites fibres électroconductrices (7) de ladite seconde couche (6, 6') forment un tissu, de préférence un tissu non tissé.

7. Matériau composite selon l'une quelconque des revendications précédentes, comprenant une première couche supplémentaire (2), et dans lequel lesdites premières couches (2) sont agencées sur des côtés opposés de ladite seconde couche (6, 6') et sont liées par induction électromagnétique par le biais d'au moins la seconde couche (6, 6').

8. Matériau composite selon l'une quelconque des revendications 1 à 6, comprenant une pluralité desdites lames (L, L') liées ensemble, dans lequel les premières couches (2) alternent avec lesdites secondes couches (6, 6') respectives et dans lequel les premières couches (2) de chaque paire de premières couches (2) consécutives sont agencées sur des côtés opposés d'une dite seconde couche (6, 6') respective et sont liées par induction électromagnétique par le biais d'au moins ladite seconde couche (6, 6').

9. Procédé de production d'un matériau composite selon l'une quelconque des revendications précédentes, dans lequel une dite première couche et une dite seconde couche (2 ; 6, 6') sont liées ensemble pour former une dite lame (L, L') de matériau composite super-soudable par moulage par compression à chaud, de préférence par moulage par compression continue, en faisant passer lesdites première et seconde couches (2 ; 6, 6') entre au moins une paire de rouleaux chauds.

10. Procédé selon la revendication 9, comprenant l'opération consistant à lier ensemble au moins deux dites lames (L, L') de matériau composite par le biais des étapes suivantes consistant à :
- étaler progressivement une première desdites lames (L, L') sur une seconde desdites lames (L, L') reposant sur un support (12) ;
- induire un courant électrique parasite au moins dans ladite seconde couche (6, 6') de ladite première lame (L, L') au moyen d'un inducteur (11) de manière à produire un chauffage localisé des zones desdites première et seconde lames (L, L') adjacentes à ladite seconde couche (6, 6') de ladite première lame (L, L') ; et
- appliquer, après ladite étape d'induction, une action de pression sur ladite première lame (L, L') pour la faire adhérer à ladite seconde lame (L, L') durant le refroidissement des lames (L, L').
